(19) Europäisches Patentamt
European Patent Office
Office européen des brevets

(11) Veröffentlichungsnummer: 0 251 998
A2

(12) **EUROPÄISCHE PATENTANMELDUNG**

(21) Anmeldenummer: 87810316.7

(22) Anmeldetag: 27.05.87

(51) Int. Cl.[4]: C 07 D 239/52
C 07 D 239/47,
C 07 D 239/42,
C 07 D 239/48,
C 07 D 239/60,
C 07 D 239/10,
C 07 D 239/95,
C 07 D 251/16,
C 07 D 251/22,
C 07 D 251/46, C 07 D 251/52

(30) Priorität: 05.06.86 CH 2291/86

(43) Veröffentlichungstag der Anmeldung: 07.01.88 Patentblatt 88/01

(84) Benannte Vertragsstaaten: BE CH DE FR GB IT LI NL

(71) Anmelder: CIBA-GEIGY AG
Klybeckstrasse 141
CH-4002 Basel (CH)

(72) Erfinder: Meyer, Willy
Talweg 49
CH-4125 Riehen (CH)

(54) **Neue Phenylsulfonylharnstoffe.**

(57) N-Phenylsulfonyl-N'-pyrimidinyl-, N'-triazolyl-oder N'-triazinylharnstoffe der Formel

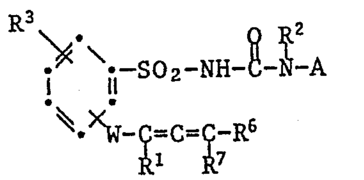

(I)

und die Salze dieser Verbindungen mit Aminen, Alkali- oder Erdalkaimetallbasen oder mit quaternären Ammoniumbasen haben gute pre-und postemergent-selektive herbizide und wuchsregulierende Eigenschaften. In dieser Formel bedeuten
$R^1$ und $R^2$ unabhängig voneinander je Wasserstoff oder $C_1$-$C_2$-Alkyl
$R^3$ Wasserstoff, Halogen, Nitro, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkoxycarbonyl, $C_1$-$C_4$-Alkythio, $C_1$-$C_4$-Alkylsulfinyl, $C_1$-$C_4$-Alkylsulfonyl, $C_2$-$C_5$-Alkoxyalkoxy oder $-SO_2NR^4R^5$
$R^4$ Wasserstoff, $C_1$-$C_2$-Alkyl oder $C_1$-$C_2$-Alkoxy
$R^5$ Wasserstoff, Methyl oder Aethyl
$R^6$ und $R^7$ je Wasserstoff, Methyl oder Aethyl
W Sauerstoff, Schwefel, eine Sulfinyl- oder Sulfonylbrücke und
A einen Rest

EP 0 251 998 A2

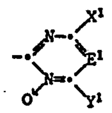 ,

A1

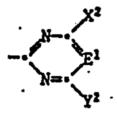 ,

A2

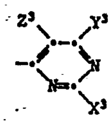 ,

A3

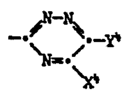 ,

A4

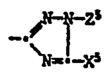 ,

A5

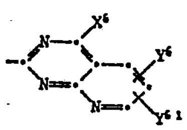 ,

A6

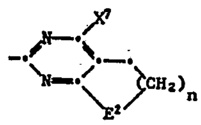 oder

A7

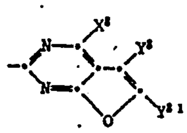

A8

bedeuten, wobei die Substituenten $E^1$, $E^2$, $X^1$, $X^2$, $X^3$, $X^4$, $X^5$, $X^6$, $X^7$, $X^8$, $Y^1$, $Y^2$, $Y^3$, $Y^4$, $Y^6$, $Y^{61}$, $Y^8$, $Y^{81}$, $Z^3$ und $Z^5$ übliche organische Reste darstellen.

**Beschreibung**

Neue Phenylsulfonylharnstoffe

Die vorliegende Erfindung betrifft neue Phenylsulfonylharnstoffe, insbesondere herbizid wirksame und pflanzenwuchsregulierende N-Phenylsulfonyl-N'-pyrimidinyl-, N'-triazolyl- oder N'-triazinylharnstoffe, Verfahren zu ihrer Herstellung, sie als Wirkstoffe enthaltende Mittel sowie deren Verwendung zum Bekämpfen von Unkräutern, vor allem selektiv in Nutzpflanzenkulturen oder zum Regulieren und Hemmen des Pflanzenwachstums.

Die erfindungsgemässen Wirkstoffe entsprechen der Formel I

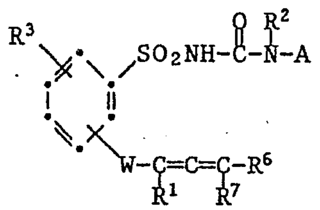

(I)

worin $R^1$ und $R^2$ unabhängig voneinander je Wasserstoff oder $C_1$-$C_2$-Alkyl,
$R^3$ Wasserstoff, Halogen, Nitro, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkoxycarbonyl, $C_1$-$C_4$-Alkythio, $C_1$-$C_4$-Alkylsulfinyl, $C_1$-$C_4$-Alkylsulfonyl oder $C_2$-$C_5$-Alkoxyalkoxy oder -$So_2NR^4R^5$
$R^4$ Wasserstoff, $C_1$-$C_2$-Alkoxy, oder $C_1$-$C_4$-Alkyl
$R^5$ Wasserstoff Methyl oder Aethyl
$R^6$ und $R^7$ je Wasserstoff, Methyl oder Aethyl
W Sauerstoff, Schwefel, eine Sulfinyl- oder Sulfonylbrücke
A einen Rest

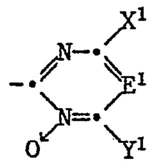 , 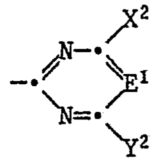 , 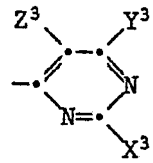 ,

A1 A2 A3

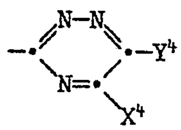 , 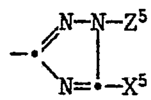 ,

A4 A5

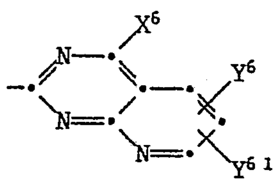 , 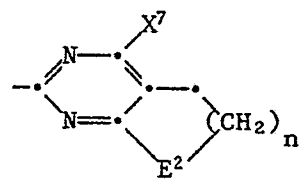 oder

A6 A7

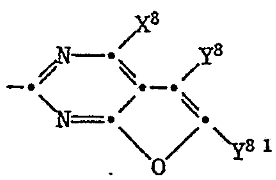

A8

bedeuten, wobei
$X^1$, $X^2$, $X^3$, $X^4$, $X^5$, $X^6$, $X^7$, $X^8$ und $Y^1$ unabhängig voneinander für Halogen, $C_1$-$C_4$-Halogenalkoxy, $C_1$-$C_4$-Halgenalkyl, $C_1$-$C_4$-Alkoxy, $C_1$-$C_4$-Alkyl, Dimethylamino, Methylamino, Aethylamino, Amino oder $C_2$-$C_4$-Alkoxyalkyl,
$E^1$ für Stickstoff oder die Methinbrücke,
$E^2$ für Sauerstoff oder die Methylenbrücke,
$Y^2$ für dasselbe wie $Y^1$ oder Cyclopropyl, Dimethoxymethyl, Diäthoxymethyl

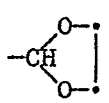 oder 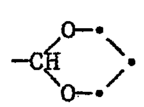 ,

$Y^3$ für Methyl, Methoxy, Aethyl, Aethoxy, $C_1$-$C_2$-Halogenalkyl oder $C_1$-$C_2$-Halogenalkoxy,
$Y^4$ für Wasserstoff, Methoxy, Aethoxy, Halogen oder $C_1$-$C_4$-Alkyl,
$Y^6$ und $Y^{61}$ unabhängig voneinander für Wasserstoff, $C_1$-$C_4$-Alkyl, Halogen, Methoxy oder Methylthio,
$Y^8$ und $Y^{81}$ unabhängig voneinander für Wasserstoff oder Methyl,
n für eins oder zwei,
$Z^3$ für Wasserstoff, Methyl, Aethyl, Methoxy, Aethoxy, Methoxycarbonyl, Aethoxycarbonyl, Halogen, Cyan, Nitro, Methylthio, Methylsulfinyl oder Methylsulfonyl,
$Z^3$ und $Y^3$ zusammen für eine $C_2$-$C_4$-Alkylenbrücke oder eine durch Sauerstoff unterbrochene $C_2$-$C_4$-Alkylenbrücke, und
$Z^5$ für Methyl oder Aethyl stehen,
sowie den Salzen dieser Verbindungen.

Harnstoffverbindungen, Triazinverbindungen und Pyrimidinverbindungen mit herbizider Wirkung sind allgemein bekannt. Kürzlich wurden Sulfonylharnstoffverbindungen mit herbizider und pflanzenwuchsregulierender Wirkung, beispielsweise in den veröffentlichten europäischen Patentanmeldungen 99339 und 107979

beschrieben.

In den Definitionen ist unter Alkyl geradkettiges oder verzweigtes Alkyl zu verstehen; z.B.: Methyl, Aethyl, n-Propyl, i-Propyl oder die vier isomeren Butyl.

Unter Alkoxy ist zu verstehen: Methoxy, Aethoxy, n-Propyloxy, i-Propyloxy oder die vier isomeren Butyloxy, insbesondere aber Methoxy, Aethoxy oder i-Propyloxy.

Beispiele für Alkylthio sind Methylthio, Aethylthio, n-Propylthio, i-Propylthio oder die vier isomeren Butylthio, insbesondere aber Methylthio und Aethylthio.

Unter Halogen selbst sowie als Teil eines Substituenten wie in Halogenalkoxy, Halogenalkylthio oder Halogenalkyl sind Fluor, Chlor und Brom, vorzugsweise jedoch Fluor und Chlor zu verstehen. Halogen alkyl selbst oder als Teil von Halogenalkoxy oder Halogenalkylthio steht in der Regel für Chlormethyl, Fluormethyl, Difluormethyl, Triflourmethyl, 2-Chloräthyl, 2,2,2-Trifluoräthyl, 1,1,2,2-Tetra-fluoräthyl, Pentafluoräthyl, 1,1,2-Trifluor-2-chloräthyl, 2,2,2-Trifluor-1,1-dichloräthyl, Pentachloräthyl, 3,3,3-Trifluorpropyl, 2,3-Dichlorpropyl, 1,1,2,3,3,3-Hexafluorpropyl, insbesondere aber Fluormethyl, Chlormethyl, Difluormethyl und Trifluormethyl.

Beispiele für Alkoxyalkyl sind: Mekthoxymethyl, Methoxyäthyl, Methoxypropyl, Aethoxyäthyl, Aethoxymethyl oder Propyloxymethyl. Beispiele für Alkoxyalkoxy sind: Methoxymethoxy, Methoxyäthoxy, Methoxypropyloxy, Aethoxymethoxy, Aethoxyäthoxy sowie Propyloxymethoxy. Alkylengruppen sind im Rahmen der Definition unter Formel I Aethylen, Propylene, Butylen, 1-Methyläthylen, 1-Aethyläthylen, 2-Methylbutylen, 1-Methylbutylen oder wenn die Brücke durch Sauerstoff unterbrochen ist auch $-CH_2-O-CH_2-$ oder $-CH_2-O-CH_2-CH_2-$.

Die Erfindung umfasst ebenfalls die Salze, die die Verbindungen der Formel I mit Aminen, Alkali- und Erdalkalimetallbasen oder quaternären Ammoniumbasen bilden können.

Unter Alkali- und Erdalkalimetallhydroxiden als Salzbildner sind die Hydroxide von Lithium, Natrium, Kalium, Magnesium oder Calcium hervorzuheben, insbesondere aber die von Natrium oder Kalium.

Beispiele für zur Salzbildung geeigneter Amine sind primäre, sekundäre und tertiäre aliphatische und aromatische Amine wie Methylamin, Aethylamin, Propylamin, i-Propylamin, die vier isomeren Butylamine, Dimethylamin, Diäthylamin, Diäthanolamin, Dipropylamin, Diisopropylamin, Di-n-butylamin, Pyrrolidin, Piperidin, Morpholin, Trimethylamin, Träthylamin, Tripropylamin, Chinuclidin, Pyridin, Chinolin und i-Chinolin, insbesondere aber Aethyl-, Propyl-, Diäthyl- oder Triäthylamin, vor allem aber iso-Propylamin, Diäthanolamin und 1,4-Diazabicyclo[2.2.2]octan.

Beispiele für quaternäre Ammoniumbasen sind im allgemeinen die Kationen von Halogenammoniumsalzen, z.B. das Tetramethylammoniumkation, das Tetraäthylammoniumkation, das Trimethyläthylammoniumkation, aber auch das Ammoniumkation.

Unter den Verbindungen der Formel I sind diejenigen bevorzugt, in denen der Rest

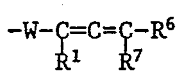

in 2-Stellung ist und A entweder
einen Pyrimidinrest der Formel

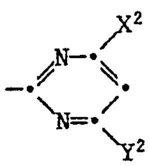

darstellt,
worin $X^2$ und $Y^2$ die unter der Formel 1 gegebene Bedeutung haben, speziell diejenigen, in denen $R^1$, $R^2$, $R^3$, $R^6$ und $R^7$ Wasserstoff bedeuten; insbesondere
N-(2-Allenyloxyphenylsulfonyl)-N'(4-methoxy-6-methylpyrimidin-2-yl)-harnstoff und
N-(2-Allenyloxyphenylsulfonyl)-N'-(4,6-dimethoxypyrimidin-2-yl)-harnstoff.
N-(2-Allenyloxyphenylsulfonyl)-N'-(4,6-bis-difluormethoxy-pyrimidin-2-yl)-harnstoff
oder einen Triazinylrest der Formel

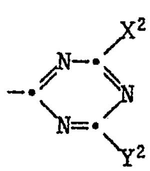

,

worin $X^2$ und $Y^2$ die unter der Formel I gegebene Bedeutung haben speziell diejenigen, in denen $R^1$, $R^2$ und $R^3$ Wasserstoff bedeuten, insbesondere

N-(2-Allenyloxyphenylsulfonyl)-N'-(4-methoxy-6-methyl-1,3,5-triazin-2-yl)-harnstoff.

Die Herstellung der Verbindungen der Formel I erfolgt im allgemeinen nach den folgenden Methoden.

Nach einem ersten Verfahren werden die Verbindungen der Formel I erhalten, indem man ein Allenyloxy-sulfonamid der Formel II

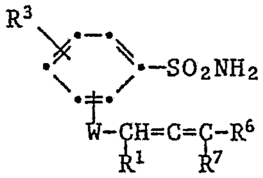

(II),

worin $R^1$, $R^3$, $R^6$, $R^7$ und W die unter Formel I gegebene Bedeutung haben, in Gegenwart einer Base mit einem Carbamat der Formel III

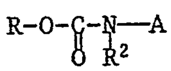

(III),

worin $R^2$, A und W die unter Formel I gegebene Bedeutung haben und R für Phenyl, Alkyl oder substituiertes Phenyl steht, umsetzt.

Nach einem zweiten Verfahren gelangt man zu Verbindungen der Formel I, indem man ein Sulfonylcarbamat der Formel IV

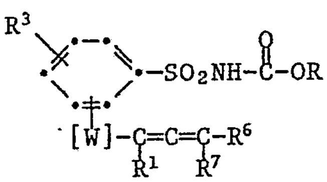

(IV)

worin $R^1$, $R^3$, $R^6$, $R^7$ und W die unter Formel I gegebene Bedeutung haben und R für Phenyl, Alkyl oder substituiertes Phenyl steht, mit einem Amin der Formel V

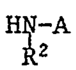

(V),

worin A und $R^2$ die unter Formel I gegebene Bedeutung haben, umsetzt.

Schliesslich kann man Verbindungen der Formel I auch erhalten, indem man ein Sulfonamid der oben gegebenen Formel II mit einem Isocyanat der Formel VI umsetzt

OCN-A VI

worin A eine der unter Formel I gegebenen Bedeutungen hat.

Die erhaltenen Harnstoffe der Formel I können gewünschtenfalls mittels Aminen, Alkalimetall- oder Erdalkalimetallhydroxiden oder quaternären Ammoniumbasen in Additionssalze überführt werden. Dieses geschieht beispielsweise durch Umsetzen mit der äquimolaren Menge Base und Verdampfen des Lösungsmittels.

Die Umsetzungen zu Verbindungen der Formel I werden vorteilhafterweise in aprotischen, inerten organischen Lösungsmitteln vorgenommen. Solche Lösungsmittel sind Kohlenwasserstoffe wie Benzol, Toluol, Xylol oder Cyclohexan, Tetrachlorkohlenstoff, oder Chorbenzol, Aether wie Diäthyläther, Aethylenglykoldimethyläther, Diäthylenglykoldimethyläther, Tetrahydrofuran oder Dioxan, Nitrile wie Acetonitril oder Propionitril, Amide wie Dimethylformamid, Diäthylformamid oder N-Methylpyrrolidinon. Die Reaktionstemperaturen liegen vorzugsweise zwischen -20° und +120°C. Die Umsetzungen der Kupplungsverfahren verlaufen im allgemeinen leicht exotherm und können bei Raumtemperatur durchgeführt werden. Zwecks Abkürzung der Reaktionszeit oder auch zum Einleiten der Umsetzung wird zweckdienlich für kurze Zeit bis zum Siedepunkt des Reaktionsgemisches aufgewärmt. Die Reaktionszeiten können ebenfalls durch Zugabe einiger Tropfen Base als Reaktionskatalysator verkürzt werden. Als Basen sind insbesondere tertiäre Amine wie Trimethylamin, Triäthylamin, Chinuclidin, 1,4-Diazabicyclo[2.2.2]-octan, 1,5-Diazabicyclo-[4.3.0]non-5-en oder 1,8-Diazabicyclo[5.4.0]undec-7-en geeignet. Als Basen können aber auch anorganische Basen wie Hydride wie Natrium-oder Calcicumhydrid, Hydroxide wie Natrium- und Kaliumhydroxid, Carbonate wie Natrium- und Kaliumcarbonat oder Hydrogencarbonate wie Kalium-und Natriumhydrogencarbonat verwendet werden.

Die Endprodukte der Formel I können durch Einengen und/oder Verdampfen des Lösungsmittels isoliert

und durch Umkristallisieren oder Zerreiben des festen Rückstandes in Lösungsmitteln, in denen sie sich nicht gut lösen, wie Aether, aromatischen Kohlenwasserstoffen oder chlorierten Kohlenwasserstoffen gereinigt werden.

Das als Zwischenprodukt benötigt Allenyloxy- und allenylthiophenylsulfonamid der Formel II wird z.B. nach einer der folgenden Methoden hergestellt: Ein Propinyloxy- resp. -thiophenylsulfonamid wird in einem inerten organischen Lösungsmittel in Gegenwart von Kalium-tert.-butylat bei einer Temperatur von 0° C-50° C verrührt bis die Propinyl-Allen-Umlagerung stattgefunden hat.

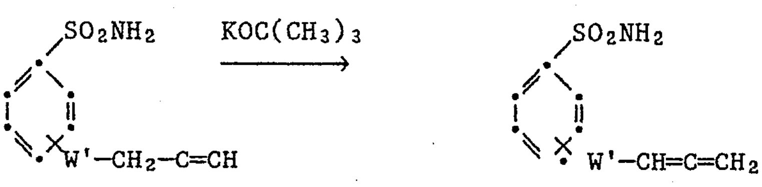


Ebenso kann man 2-Chlorallyloxy resp. thio-penylsulfonamid in Gegenwart von Kalium-tert.-butylat zum Allenyloxy-phenylsulfonamid umsetzten

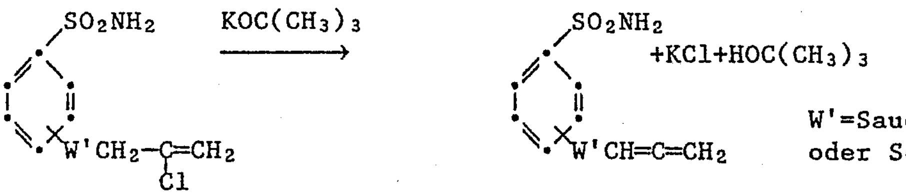


W'=Sauerstoff oder Schwefel

Die Umsetzungen resp. Umlagerungen finden bei niedrigen Temperaturen von 0-50°C in einem inerten organischen Lösungsmittel wie Dimethylsulfoxid, Dimethylformamid, Dioxan, Cellosolve, Dipropyläther Aethanol oder Tetrahydrofuran statt. Nach Beendigung der Reaktion wird das Reaktionsgemisch mit verdünnter wässriger Mineralsäure neutral gestellt und das Allen durch Extraktion daraus isoliert.

Sulfoxide und Sulfone der den Allenylthiosulfonamide erhält man durch Oxidation mit Wasserstoffperoxid in organischen Lösungmitteln wie z.B. Essigsäure gemäss dem folgenden Schema:

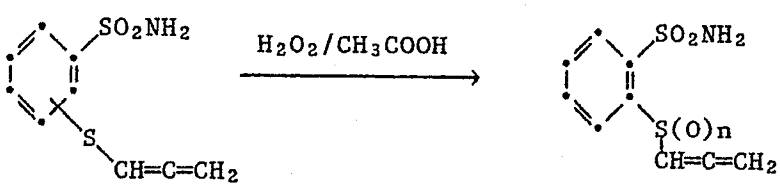


Die Zwischenprodukte der Formel IV werden nach bekannten Methoden aus dem Allenyloxyphenylsulfonamid der Formel II erhalten. Die als Ausgangsmaterialien verwendeten Aminopyrimidine, Aminotriazole und Aminotriazine der Formel V sowie entsprechende Carbamate der Formel III sind entweder bekannt oder sie lassen sich nach bekannten und in der Literatur beschriebenen Methoden erhalten. Die Wirkstoffe der Formel I sind stabile Verbindungen. Ihre Handhabung bedarf keiner vorsorglichen Massnahmen.

Bei geringeren Aufwandmengen zeichnen sich die Verbindungen der Formel I durch gute selektiv-wuchshemmende und selektiv-herbizide Eigenschaften aus, die sie ausgezeichnet zum Einsatz in Kulturen von Nutspflanzen, insbesondere in Getreide, Reis, Baumwolle, Soja und Mais befähigen. Es werden dabei teilweise auch Unkräuter geschädigt, welchen bisher nur mit Totalherbiziden beizukommen war.

Die Wirkungsart dieser Wirkstoffe ist unüblich. Viele sind translozierbar, d.h. sie werden von der Pflanze aufgenommen und an andere Stellen transportiert, wo sie dann zur Wirkung kommen. So gelingt es beispielsweise, durch Oberflächenbehandlung perennierende Unkräuter bis in die Wurzeln zu schädigen. Die neuen Verbindungen der Formel I wirken bereits bei - im Vergleich zu anderen Herbiziden und Wuchsregulatoren - sehr geringen Aufwandmengen.

Die Verbindungen der Formel I haben ausserdem starke pflanzenwuchsregulierende, insbesondere pflanzenwuchshemmende, Eigenschaften. Es werden sowohl Monokotyledonen als auch Dikotyledonen in ihrem Wachstum beeinträchtigt.

Ein weiterer Mechanismus der Ertragssteigerung mit Wachstumshemmern beruht darauf, dass die Nährstoffe in stärkerem Masse der Blüten-und Fruchtbildung zugute kommen, während das vegetative Wachstum eingeschränkt wird.

Bei grösseren Aufwandmengen an Wirkstoffen der Formel I werden alle getesteten Pflanzen in ihrer

Entwicklung so geschädigt, dass sie absterben.

Die Erfindung betrifft auch herbizide und pflanzenwachstumsregulierende Mittel, welche einen neuen Wirkstoff der Formel I enthalten, sowie Verfahren zur pre- und post-emergenten Unkrautbekämpfung und zur Hemmung des Pflanzenwuchses von monokotylen und dikotylen Pflanzen, insbesondere Gräsern, tropischen Bodenbedeckern und Tabakgeiztrieben.

Die Verbindungen der Formel I werden in unveränderter Form oder vorzugsweise als Mittel zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weis verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise bergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C^8$ bis $C^{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Aether und Ester, wie Aethanol, Aethylenglykol, Aethylenglykolmonomethyl- oder -äthyl äther, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation-und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C^{10}$-$C^{22}$), wie z.B. die Na- oder K-Salze der Oel- oder Stearinsäure, oder von natürlichen Fettsäuregemischen, die z.B. aus Kokosnuss- oder Talgöl gewonnen werden können, genannt. Ferner sind auch die Fettsäuremethyl-taurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2-Sulfonsäuregruppen und einen Fettsäurerest mit 8 bis 22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Aethylenoxid-Adduktes oder Phospholipide in Frage.

Als nicht ionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 10 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykoläthergruppen enthaltenden Polyäthylenoxidaddukte an Polypropylenglykol, Aethylendiaminopolypropylenglykol und Alkylpolypropylenglykol mit 1 bis 10 Kohlenstoffatomen in der Alkylkette, die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglykoläther, Polypropylen-Polyäthylenoxi-d-addukte, Tributylphenoxypolyäthoxyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitan-trioleat in Betracht.

Bei den kationischen Tensiden handelt es sich von allem um quaternäre Ammoniumsalze, welche als N-Substituenten mindestens einen Alkylrest mit 8 bis 22 C-Atomen enthalten und als weitere Substituenten niederige, gegebenenfalls halogenierte Alkyl-, Benzyl-oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyldi(2-chloräthyl)-äthylammoniumbromid.

Die in der Formulierungstechnik gebräuchlichen Tenside sind u.a. in folgenden Publikationen beschrieben:
"Mc Cutcheon"s Detergents and Emulsifiers Annual" MC Publishing Corp., Ridgewood, New Jersey, 1981;
H. Stache, "Tensid-Taschenbuch", 2. Aufl., C. Hanser Verlag, München, Wien, 1981;
M. and J. Ash. "Encyclopedia of Surfactants", Vol. I-III, Chemical Publishing Co., New York, 1980-1981.

Die pestiziden Zubereitungen enthalten in der Regel 0,1 bis 95 %, insbesondere 0,1 bis 80 % Wirkstoff der Formel I, 1 bis 99,9 % eines festen oder flüssigen Zusatzstoffes und 0 bis 25 %, insbesondere 0,1 bis 25 % eines Tensides.

Insbesondere setzen sich bevorzugte Formulierungen folgendermassen zusammen: (% = Gewichtsprozent).

<u>Emulgierbare Konzentrate</u>:
Aktiver Wirkstoff: 1 bis 20 %, bevorzugt 5 bis 10 %
oberflächenaktive Mittel: 5 bis 30 %, vorzugsweise 10 bis 20 %
flüssiges Trägermittel: 50 bis 94 %, vorzugsweise 70 bis 85 %

<u>Stäube</u>:
Aktiver Wirkstoff: 0,1 bis 10 %, vorzugsweise 0,1 bis 1 %
festes Trägermittel: 99,9 bis 90 %, vorzugsweise 99,9 bis 99 %

<u>Suspensions-Konzentrate</u>:
Aktiver Wirkstoff: 5 bis 75 %, vorzugsweise 10 bis 50 %
Wasser: 94 bis 24 %, vorzugsweise 88 bis 30 %
oberflächenaktives Mittel: 1 bis 40 %, vorzugsweise 2 bis 30 %

<u>Benetzbares Pulver</u>::
Aktiver Wirkstoff: 0,5 bis 90 %, vorzugweise 1 bis 80 %
oberflächenaktives Mittel: 0,5 bis 20 %, vorzugsweise 1 bis 15 %
festes Trägermittel: 5 bis 95 %, vorzugsweise 15 bis 90 %

<u>Granulate</u>:
Aktiver Wirkstoff: 0,5 bis 30 %, vorzugsweise 3 bis 15 %
festes Trägermittel: 99,5 bis 70 %, vorzugsweise 97 bis 85 %.

Während als Handelsware eher konzentrierte Mittel bevorzugt werden, verwendet der Endverbraucher in der Regel verdünnte Mittel. Die Anwendungsformen können bis hinab zu 0,001 % an Wirkstoff verdünnt werden. Die Aufwandmengen betragen in der Regel 0,001 bis 10 kg AS/ha, vorzugsweise 0,025 bis 5 kg AS/ha.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

<u>Herstellungsbeispiele</u>

<u>Beispiel 1</u>: Herstellung von
N-(2-Allenyloxy-phenylsulfonyl)-N'-4,6-bis-difluormethoxy-1,3-pyrimid-2-yl)-harnstoff.

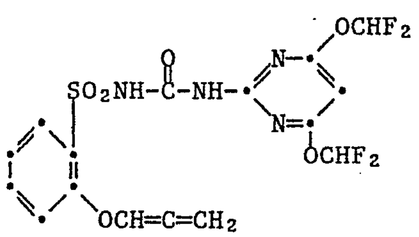

Eine Mischung von 2,53 g 4,6-Bis-difluormethoxy-1,3-pyrimidin-2-isocyanat, 2,11 g 2-Allenyloxy-phenylsulfonamid und 70 ml Methylenchlorid wird tropfenweise mit einer Lösung von 1,52 g 1,8-Diazabicyclo-[5.4.0]-undec-7-en-(1.5-5) und 10 ml Methylenchlorid versetzt. Nach 5 Stunden rühren bei Raumtemperatur wird vollständig eingedampft. Der Rückstand wird mit Wasser verrührt und mit verdünnter Salzsäure auf pH 6 gestellt. Durch Extrahieren mit Essigester, Waschen, Trocknen, Eindampfen und Kristallisieren aus Aether erhält man 0,65 g der Titelverbindung vom Schmelzpunkt 139-40°.

Das als Ausgangsprodukt benötigte 2-Allenyloxy-phenylsulfonamid wird wie folgt erhalten:

a) 2-Allenloxy-phenylsulfonamid

Eine Lösung von 5 g 2-(2-Chlorprop-2-enyloxy)-phenylsulfonamid und 30 ml Dimethylsulfoxyd wird portionenweise mit 4,5 g Kalium-tert.-butylat versetzt, so dass die Temperatur nicht über 28° C steigt. Nach 20 Stunden rühren bei 20-25° C wird das Reaktionsgemisch mit Eiswasser versetzt und durch Zutropfen von 10%iger Salzsäure auf pH 7 gestellt. Durch Extrahieren mit Essigester, Waschen der organischen Phase mit Wasser und Salzsäure, Trocknen über Natriumsulfat und Eindampfen und Chromatographieren des Rückstandes an einer Kieselgel-Säule erhält man schliesslich reines 2-Allenyloxyphenylsulfonamid vom Schmelzpunkt 94-95° C.

Deises Produkt kann auch folgendermassen hergestellt werden:

b) 2-Allenyloxy-phenylsulfonamid

Eine Lösung von 6,33 g 2-Propinyloxy-phenylsulfonamid und 40 ml Dimethylsulfoxyd wird portionenweise mit 4,4 g Kalium-tert.-butylat versetzt, so dass die Temperatur nicht über 28° C steigt. Nach 4 Stunden rühren bei 20-25° C wird das Reaktionsgemisch mit Eiswasser versetzt und durch Zutropfen von verdünnter Salzsäure auf pH 7 gestellt. Durch Extrahieren mit Essigester, Waschen der organischen Phase mit Wasser und Sole, Trocknen über Natriumsulfat und Eindampfen und Chromatograhieren des Rückstandes an einer Kieselgel-Säule erhält man 1 g 2-Allenyloxy-phenylsulfonamid vom Schmelzpunkt 94-95° C.

Beispiel 2: Herstellung von N-(2-Allenyloxy-penyl-sulfonyl)-N'-(4-methoxy-6-methyl 1,3,5-triazin-2-yl)-harnstoff.

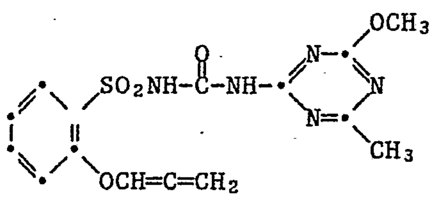

Eine Mischung von 1,7 g 2-Allenyloxyphenylsulfonamid, 2,08 g 4-Methoxy-6-methyl-1,3,5-triazin-2-yl-phenylcarbamat, 1,22 g 1,8-Diazabicyclo-[5.4.0]-undec-7-en-(1.5-5) und 50 ml absolutes Dioxan wird 4 Stunden bei 20-25° C gerührt. Durch Eingiessen in Wasser, Zutropfen von 10%iger Salzsäure bis pH 6, Ausschütteln mit Essigester, Trocknen der organischen Phase, Eindampfen und Kristallisieren aus Essigester erhält man 1,8 g der Titelverbindung vom Schmelzpunkt 150-51° C Zers.

In analoger Weise zu diesen Beispielen werden die in den Tabellen 1-10 aufgeführten Verbindungen hergestellt:

Tabelle 01

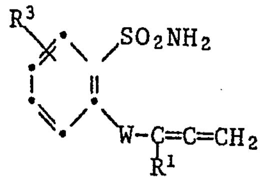

| Verb-Nr. | $R^1$ | $R^3$ | W | Smp.(°C) |
|---|---|---|---|---|
| 01.01 | H | H | O | 94-95°C |
| 01.02 | $CH_3$ | H | O | 110-111°C |
| 01.03 | H | 5-$CH_3$ | O | |
| 01.04 | H | 5-Cl | O | |
| 01.05 | H | 5-F | O | |
| 01.06 | H | 6-Cl | O | |
| 01.07 | H | 6-$NO_2$ | O | |
| 01.08 | H | 3-Cl | O | |
| 01.09 | H | H | S | 119-120°C |
| 01.10 | H | H | SO | |
| 01.11 | H | H | $SO_2$ | 169-70°C |
| 01.12 | $CH_3$ | H | S | |
| 01.13 | $CH_3$ | H | SO | |
| 01.14 | $CH_3$ | H | $SO_2$ | |
| 01.15 | 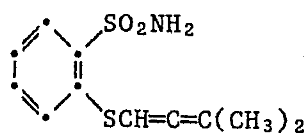 | | | 102-103°C |

Tabelle 02

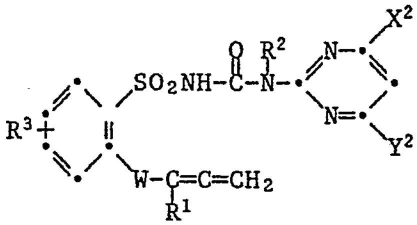

(Ib)

| Verb-Nr. | $R^1$ | $R^2$ | $R^3$ | W | $X^2$ | $Y^2$ | Smp.(°C) |
|---|---|---|---|---|---|---|---|
| 02.01 | H | H | H | O | $CH_3$ | $CH_3$ | 179-180°C |
| 02.02 | H | H | H | O | $CH_3$ | $C_2H_5$ | |
| 02.03 | H | H | H | O | $CH_3$ | $OCH_3$ | 145-146°C |
| 02.04 | H | H | H | O | $CH_3$ | $OC_2H_5$ | |
| 02.05 | H | H | H | O | $CH_3$ | $OCHF_2$ | 157-158°C Zers. |
| 02.06 | H | H | H | O | $CH_3$ | $OCH_2CF_3$ | |
| 02.07 | H | H | H | O | $CH_3$ | 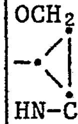 | |
| 02.08 | H | H | H | O | $CH_3$ | $HN-CH_3$ | |
| 02.09 | H | H | H | O | $CH_3$ | $N(CH_3)_2$ | |
| 02.10 | H | H | H | O | $CH_2Cl$ | $OCH_3$ | |
| 02.11 | H | H | H | O | $C_2H_5$ | $OCH_3$ | |
| 02.12 | H | H | H | O | $C_2H_5$ | $OCHF_2$ | |
| 02.13 | H | H | H | O | $C_2H_5$ | $HN-CH_3$ | |
| 02.14 | H | H | H | O | $OCH_3$ | $OCH_3$ | 180-181°C |
| 02.15 | H | H | H | O | $OCH_3$ | $OC_2H_5$ | |
| 02.16 | H | H | H | O | $OCH_3$ | $OCHF_2$ | |
| 02.17 | H | H | H | O | $OCH_3$ | $OCH_2CF_3$ | |
| 02.18 | H | H | H | O | $OCH_3$ | 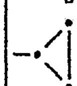 | |
| 02.19 | H | H | H | O | $OCH_3$ | $N(CH_3)_2$ | |
| 02.20 | H | H | H | O | $OCH_3$ | Cl | |
| 02.21 | H | H | H | O | $OCH_3$ | $CH_2F$ | |

Tabelle 02 (Fortsetzung)

| Verb-Nr. | $R^1$ | $R^2$ | $R_3$ | W | $X^2$ | $Y^2$ | Smp.(°C) |
|---|---|---|---|---|---|---|---|
| 02.22 | H | H | H | O | $OC_2H_5$ | $HNCH_3$ | |
| 02.23 | H | H | H | O | $OCHF_2$ | $OCHF_2$ | 139-140°C |
| 02.24 | H | H | H | O | $CH_2OCH_3$ | $OCH_3$ | |
| 02.25 | $CH_3$ | H | H | O | $CH_3$ | $CH_3$ | |
| 02.26 | $CH_3$ | H | H | O | $CH_3$ | $OCH_3$ | 165-166°C |
| 02.27 | $CH_3$ | H | H | O | $OCH_3$ | $OCH_3$ | 200-201°C |
| 02.28 | $CH_3$ | H | H | O | $OCH_3$ | $OCHF_2$ | |
| 02.29 | $CH_3$ | H | H | O | $OCHF_2$ | $OCHF_2$ | |
| 02.30 | $CH_3$ | $CH_3$ | H | O | $CH_3$ | $OCH_3$ | |
| 02.31 | $CH_3$ | $CH_3$ | H | O | $OCH_3$ | $OCH_3$ | |
| 02.32 | H | $CH_3$ | H | O | $OCH_3$ | $OCHF_2$ | |
| 02.33 | H | $CH_3$ | H | O | $OCHF_2$ | $OCHF_2$ | |
| 02.34 | H | $CH_3$ | H | O | $CH_3$ | $OCH_3$ | |
| 02.35 | H | $CH_3$ | H | O | $OCH_3$ | $OCH_3$ | |
| 02.36 | $CH_3$ | H | H | O | $CH_3$ | $OCHF_2$ | |
| 02.37 | H | H | $6-NO_2$ | O | $OCH_3$ | $OCH_3$ | |
| 02.38 | H | H | 5-Cl | O | $OCH_3$ | $OCH_3$ | |
| 02.39 | H | H | 5-F | O | $OCH_3$ | $OCH_3$ | |
| 02.40 | H | H | $5-CH_3$ | O | $CH_3$ | $OCH_3$ | |
| 02.41 | H | H | 3-Cl | O | $CH_3$ | $OCH_3$ | |
| 02.42 | H | H | 5-Cl | O | $CH^3$ | $OCH_3$ | |
| 02-43 | H | H | 6-Cl | O | $CH_3$ | $OCH_3$ | |
| 02.44 | H | H | 5-F | O | $CH_3$ | $OCH_3$ | |

Tabelle 02 (Fortsetzung)

| Verb-Nr. | $R^1$ | $R^2$ | $R^3$ | W | $X^2$ | $Y^2$ | Smp. (°C) |
|---|---|---|---|---|---|---|---|
| 02.45 | H | H | H | S | $CH_3$ | $CH_3$ | |
| 02.46 | H | H | H | S | $CH_3$ | $C_2H_5$ | |
| 02.47 | H | H | H | S | $CH_3$ | $OCH_3$ | |
| 02.48 | H | H | H | S | $CH_3$ | $OC_2H_5$ | |
| 02.49 | H | H | H | S | $CH_3$ | $OCHF_2$ | |
| 02.50 | H | H | H | S | $CH_3$ | $OCH_2CF_3$ | |
| 02.51 | H | H | H | S | $CH_3$ | 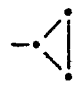 | |
| 02.52 | H | H | H | S | $CH_3$ | $HN-CH_3$ | |
| 02.53 | H | H | H | S | $CH_3$ | $N(CH_3)_2$ | |
| 02.54 | H | H | H | S | $CH_2Cl$ | $OCH_3$ | |
| 02.55 | H | H | H | S | $C_2H_5$ | $OCH_3$ | |
| 02.56 | H | H | H | S | $C_2H_5$ | $OCHF_2$ | |
| 02.57 | H | H | H | S | $C_2H_5$ | $HN-CH_3$ | |
| 02.58 | H | H | H | S | $OCH_3$ | $OCH_3$ | 156-157°C |
| 02.59 | H | H | H | S | $OCH_3$ | $OCHF_2$ | |
| 02.60 | H | H | H | S | $OCH_3$ | $OCH_2CF_3$ | |
| 02.61 | H | H | H | S | $OCH_3$ | 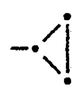 | |
| 02.62 | H | H | H | S | $OCH_3$ | $N(CH_3)_2$ | |
| 02.63 | H | H | H | S | $OCH_3$ | Cl | |
| 02.64 | H | H | H | S | $OCH_3$ | $CH_2F$ | |
| 02.65 | H | H | H | S | $OC_2H_5$ | $HN-CH_3$ | |
| 02.66 | H | H | H | S | $OCHF_2$ | $OCHF_2$ | |

Tabelle 02 (Fortsetzung)

| Verb-Nr. | $R^1$ | $R^2$ | $R^3$ | W | $X^2$ | $Y^2$ | Smp. (°C) |
|---|---|---|---|---|---|---|---|
| 02.67 | $CH_3$ | H | H | S | $CH_3$ | $CH_3$ | |
| 02.68 | $CH_3$ | H | H | S | $OCH_3$ | $OCH_3$ | 168-169°C |
| 02.69 | H | H | H | SO | $CH_3$ | $OCH_3$ | |
| 02.70 | H | H | H | SO | $OCH_3$ | $OCH_3$ | |
| 02.71 | H | H | H | $SO_2$ | $CH_3$ | $OCH_3$ | |
| 02.72 | H | H | H | $SO_2$ | $OCH_3$ | $OCH_3$ | 181-182°C |
| 02.73 | H | H | H | $SO_2$ | $OCHF_2$ | $OCHF_2$ | |
| 02.74 | H | H | H | $SO_2$ | $CH_3$ | $CH_3$ | |
| 02.75 | H | H | H | $SO_2$ | $CH_3$ | $OCHF_2$ | |
| 02.76 | H | H | H | O | $OC_2H_5$ | $OCHF_2$ | |
| 02.77 | H | H | H | S | $OC_2H_5$ | $OCHF_2$ | |
| 02.78 | H | H | H | $SO_2$ | $OCH_3$ | $OCHF_2$ | |

Tabelle 03

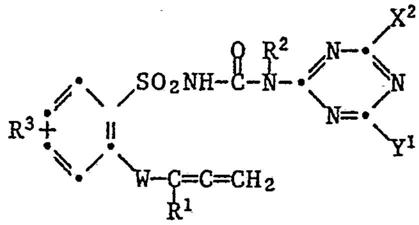

(Ic)

| Verb-Nr. | $R^1$ | $R^2$ | $R^3$ | W | $X^2$ | $Y^2$ | Smp.(°C) |
|---|---|---|---|---|---|---|---|
| 03.01 | H | H | H | O | $CH_3$ | $OCH_3$ | 150-151°C Zers. |
| 03.02 | H | H | H | O | $CH_3$ | $OC_2H_5$ | |
| 03.03 | H | H | H | O | $CH_3$ | $OCH_2CF_3$ | |
| 03.04 | H | H | H | O | $CH_3$ | $CH_3$ | |
| 03.05 | H | H | H | O | $CH_3$ | 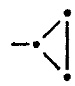 | |
| 03.06 | H | H | H | O | $OCH_3$ | $OCH_3$ | 154-155°C |
| 03.07 | H | H | H | O | $OCH_3$ | $OC_2H_5$ | |
| 03.08 | H | H | H | O | $OCH_3$ | $N(CH_3)_2$ | |
| 03.09 | H | H | H | O | $OCH_3$ | $OCH_2CF_3$ | |
| 03.10 | H | H | H | O | $CH_2Cl$ | $OCH_3$ | |
| 03.11 | H | H | H | O | $CH_2F$ | $OCH_3$ | |
| 03.12 | H | H | H | O | $C_2H_5$ | $OCH_3$ | |
| 03.13 | H | H | H | O | $OC_2H_5$ | $HN-CH_3$ | |
| 03.14 | H | H | H | O | $OC_2H_5$ | $OC_2H_5$ | |
| 03.15 | $CH_3$ | H | H | O | $CH_3$ | $OCH_3$ | 146-147°C |
| 03.16 | $CH_3$ | H | H | O | $OCH_3$ | $OCH_3$ | 150-152°C |
| 03.17 | $CH_3$ | $CH_3$ | H | O | $CH_3$ | $OCH_3$ | |
| 03.18 | H | H | $5-CH_3$ | O | $CH_3$ | $OCH_3$ | 162-163°C |
| 03.19 | H | H | $5-CH_3$ | O | $CH_3$ | $OCH_3$ | |

Tabelle 03 (Fortsetzung)

| Verb-Nr. | $R^1$ | $R^2$ | $R^3$ | W | $X^2$ | $Y^2$ | Smp. (°C) |
|---|---|---|---|---|---|---|---|
| 03.20 | H | H | H | S | $CH_3$ | $OCH_3$ | 130-131°C |
| 03.21 | H | H | H | S | $OCH_3$ | $OCH_3$ | 144-145°C |
| 03.22 | H | H | H | S | $OCH_3$ | $N(CH_3)_2$ | |
| 03.23 | H | H | H | S | $OCH_3$ | $OCH_2CF_3$ | |
| 03.24 | H | H | H | S | $CH_3$ | $OC_2H_5$ | |
| 03.25 | H | H | H | S | $C_2H_5$ | $OCH_3$ | |
| 03.26 | H | H | H | S | $OC_2H_5$ | $HN-CH_3$ | |
| 03.27 | $CH_3$ | H | H | S | $CH_3$ | $OCH_3$ | 156-157°C |
| 03.28 | $CH_3$ | H | H | S | $OCH_3$ | $OCH_3$ | |
| 03.29 | H | H | H | SO | $CH_3$ | $OCH_3$ | |
| 03.30 | H | H | H | SO | $OCH_3$ | $OCH_3$ | |
| 03.31 | $CH_3$ | H | H | $SO_2$ | $CH_3$ | $OCH_3$ | |
| 03.32 | $CH_3$ | H | H | $SO_2$ | $OCH_3$ | $OCH_3$ | |
| 03.33 | 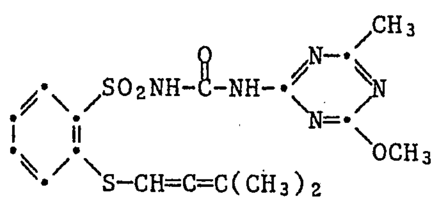 | | | | | | 150-151°C |

Tabelle 04

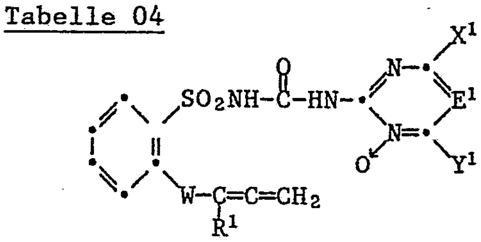

(Ia)

| Verb-Nr. | $R^1$ | W | $E^1$ | $X^1$ | $Y^1$ | Smp.(°C) |
|---|---|---|---|---|---|---|
| 04.01 | H | O | CH | $CH_3$ | $OCH_3$ | |
| 04.02 | $CH_3$ | O | CH | $CH_3$ | $OCH_3$ | |
| 04.03 | H | S | CH | $CH_3$ | $OCH_3$ | |
| 04.04 | H | O | CH | $CH_3$ | $CH_3$ | |
| 04.05 | $CH_3$ | O | CH | $CH_3$ | $CH_3$ | |
| 04.06 | H | S | CH | $CH_3$ | $CH_3$ | |
| 04.07 | H | O | N | $CH_3$ | $CH_3$ | |
| 04.08 | $CH_3$ | O | N | $CH_3$ | $CH_3$ | |
| 04.09 | H | S | N | $CH_3$ | $CH_3$ | |
| 04.10 | H | O | CH | $CH_3$ | $OC_2H_5$ | |
| 04.11 | H | O | CH | $OCH_3$ | $OCH_3$ | |
| 04.12 | H | O | CH | $N(CH_3)_2$ | $OCH_3$ | |
| 04.13 | H | $SO_2$ | CH | $CH_3$ | $OCH_3$ | |
| 04.14 | H | $SO_2$ | N | $CH_3$ | $OCH_3$ | |

Tabelle 05

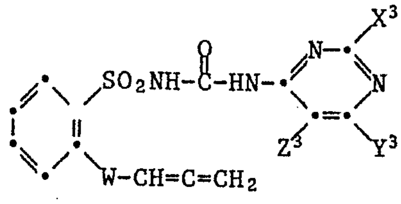

(Ia)

| Verb-Nr. | $X^3$ | $Y^3$ | $Z^3$ | W | Smp (°C) |
|---|---|---|---|---|---|
| 05.01 | $OCH_3$ | $OCH_3$ | $CH_3$ | O | |
| 05.02 | $CH_3$ | $OCH_3$ | $CH_3$ | O | |
| 05.03 | $OCH_3$ | $CH_3$ | $CH_3$ | O | |
| 05.04 | $OCH_3$ | $OCH_3$ | Cl | O | |
| 05.05 | $CH_3$ | $OCH_3$ | Cl | O | |
| 05.06 | $OCH_3$ | $OCH_3$ | F | O | |
| 05.07 | $OCH_3$ | $OCH_3$ | $CF_3$ | O | |
| 05.08 | $OCH_3$ | $-CH_2-CH_2-CH_2-$ | | O | |
| 05.09 | $OCH_3$ | $OCH_3$ | $CH_3$ | S | |
| 05.10 | $OCH_3$ | $OCH_3$ | $OCH_3$ | $SO_2$ | |

Tabelle 06

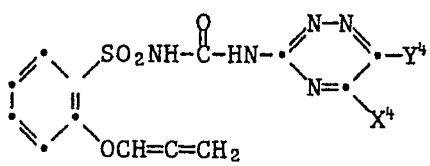

(Ie)

| Verb-Nr. | $X^4$ | $Y^4$ | Smp.(°C) |
|---|---|---|---|
| 06.01 | $CH_3$ | $CH_3$ | |
| 06.02 | $CH_3$ | H | |
| 06.03 | $OCH_3$ | H | |
| 06.04 | $OCH_3$ | $OCH_3$ | |

Tabelle 07

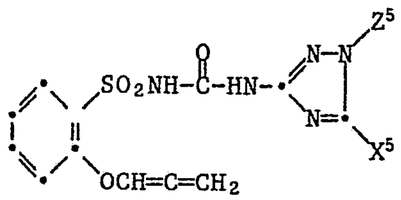

(If)

| Verb-Nr. | $X^5$ | $Z^5$ | Smp.(°C) |
|---|---|---|---|
| 07.01 | $OCH_3$ | $CH_3$ | |
| 07.02 | $CH_3$ | $CH_3$ | |

Tabelle 08

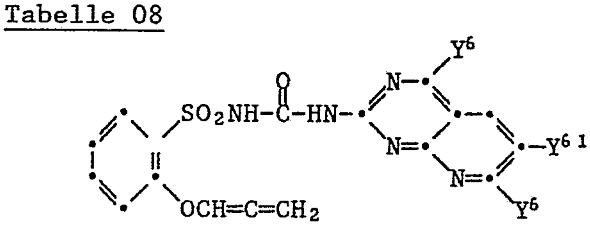

(Ig)

| Verb-Nr. | $X^6$ | $Y^6$ | $Y^{61}$ | Smp.(°C) |
|---|---|---|---|---|
| 08.01 | $OCH_3$ | $CH_3$ | H | |
| 08.02 | $OCH_3$ | H | $CH_3$ | |
| 08.03 | $CH_3$ | $CH_3$ | H | |

Tabelle 09

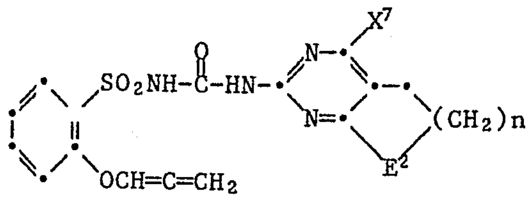

(Ih)

| Verb-Nr. | $E^2$ | n | $X^7$ | Smp.(°C) |
|---|---|---|---|---|
| 09.01 | O | 1 | $CH_3$ | |
| 09.02 | O | 1 | $OCH_3$ | |
| 09.03 | $CH_2$ | 1 | $OCH_3$ | |
| 09.04 | O | 2 | $OCH_3$ | |
| 09.05 | $CH_2$ | 2 | $OCH_3$ | |
| 09.06 | $CH_2$ | 1 | $CH_3$ | |

Tabelle 10

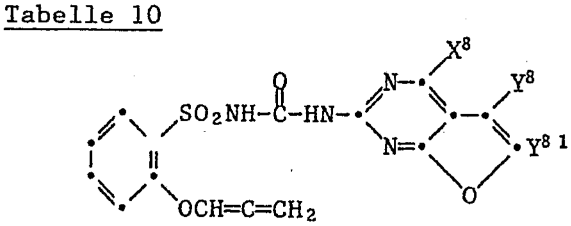

(Ii)

| Verb-Nr. | $X^8$ | $Y^8$ | $Y^{81}$ | Smp.(°C) |
|---|---|---|---|---|
| 10.01 | $CH_3$ | H | $CH_3$ | |
| 10.02 | $OCH_3$ | H | $CH_3$ | |
| 10.03 | $OCH_3$ | H | H | |
| 10.04 | $CH_3$ | H | $CH_3$ | |
| 10.05 | $OCH_3$ | $CH_3$ | $CH_3$ | |

Formulierungsbeispiele:

Beispiel 3: Formulierungsbeispiele für Wirkstoffe der Formel I (% = Gewichtsprozent)

a) Spritzpulver

| | a) | b) | c) |
|---|---|---|---|
| Wirkstoff der Tabelle 2 | 20 % | 50 % | 0,5 % |
| Na-Ligninsulfonat | 5 % | 5 % | 5 % |
| Na-Laurylsulfat | 3 % | - | - |
| Na-Diisobutylnaphthalinsulfonat | - | 6 % | 6 % |
| Octylphenolpolyäthylenglykoläther (7-8 Mol AeO) | - | 2 % | 2 % |
| Hochdisperse Kieselsäure | 5 % | 27 % | 27 % |
| Kaolin | 67 % | - | - |
| Natriumchlorid | - | - | 59,5 % |

Die Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

b) Emulsions-Konzentrat

| | a) | b) |
|---|---|---|
| Wirkstoff der Tabelle 3 | 10 % | 1 % |
| Octylphenolpolyäthylenglykoläther (4-5 Mol AeO) | 3 % | 3 % |
| Ca-Dodecylbenzolsulfonat | 3 % | 3 % |
| Ricinusölpolyglykoläther (36 Mol AeO) | 4 % | 4 % |
| Cyclohexanon | 30 % | 10 % |
| Xylolgemisch | 50 % | 79 % |

Aus diesem Konzentrat können durch Verdünnen mit Wasser Emulsionen jeder gewünschten Konzentration hergestellt werden.

c) Stäubemittel

| | a) | b) |
|---|---|---|
| Wirkstoff der Tabelle 2 | 0,1 % | 1 % |
| Talkum | 99,9 % | - |
| Kaolin | - | 99 % |

Man erhält anwendungsfertige Stäubemittel, indem der Wirkstoff mit dem Träger vermischt und auf einer geeigneten Mühle vermahlen wird.

d) Extruder Granulat

| | a) | b) |
|---|---|---|
| Wirkstoff der Tabelle 2 | 10 % | 1 % |
| Na-Ligninsulfonat | 2 % | 2 % |
| Carboxymethylcellulose | 1 % | 1 % |
| Kaolin | 87 % | 96 % |

Der Wirkstoff wird mit den Zusatzstoffen vermischt, vermahlen und mit Wasser angefeuchtet. Dieses Gemisch wird extrudiert und anschliessend im Lufstrom getrocknet.

e) Umhüllungs-Granulat

| | |
|---|---|
| Wirkstoff der Tabelle 3 | 3 % |
| Polyäthylenglykol (MG 200) | 3 % |
| Kaolin | 94 % |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

f) Suspensions-Konzentrat

| | a) | b) |
|---|---|---|
| Wirkstoff des Beispiels 1 | 40 % | 5 % |
| Aethylenglykol | 10 % | 10 % |
| Nonylphenolpolyäthylenglykoläther (15 Mol AeO) | 6 % | 1 % |
| Na-Ligninsulfonat | 10 % | 5 % |
| Carboxymethylcellulose | 1 % | 1 % |
| 37 %ige wässrige Formaldehyd-Lösung | 0,2 % | 0,2 % |
| Silikonöl in Form einer 75 %igen wässrigen Emulsion | 0,8 % | 0,8 % |
| Wasser | 32 % | 77 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

g) Salzlösung

| | |
|---|---|
| Wirkstoff des Beispiels 2 | 5 % |
| Isopropylamin | 1 % |
| Octylphenolpolyäthylenglykoläther (78 Mol AeO) | 3 % |
| Wasser | 91 % |

Biologische Beispiele:

Beispiel 4: Herbizidwirkung vor dem Auflaufen der Pflanzen

Kunststofftöpfe werden mit expandiertem Vermiculit (Dichte: 0,135 g/cm³, Wasserabsorptionsvermögen: 0,565 1/1) gefüllt. Nach dem Sättigen des nicht adsorptiven Vermiculits mit einer wässrigen Wirkstoffemulsion in deionisiertem Wasser, die die Wirkstoffe in einer Konzentration von 70,8 ppm enthält, werden Samen der folgenden Pflanzen auf die Oberfläche gesät: Nasturtium officinalis, Agrostis tenuis, Stellaria media und Digitaria sanguinalis. Die Versuchsgefässe werden anschliessend in einer Klimakammer bei 20°C, einer Beleuchtung von ca. 20 kLux und einer relativen Luftfeuchtigkeit von 70 % gehalten. Während einer Keimphase von 4 bis 5 Tagen werden die Töpfe zur Erhöhung der örtlichen Luftfeuchtigkeit mit lichtdurchlässigem Material abgedeckt und mit deionisiertem Wasser gegossen. Nach dem 5.Tag wird dem Giesswasser 0,5 % eines handelsüblichen Flüssigdüngers (®Greenzit, ex Ciba-Geigy) zugesetzt. 12 Tage nach der Aussaat wird der Versuch ausgewertet und die Wirkung auf die Versuchspflanzen nach dem folgenden Massstab bewertet:

1 : Pflanze nicht gekeimt oder total abgestorben
2-3: sehr starke Wirkung
4-6: mittlere Wirkung
7-8: schwache Wirkung
9: keine Wirkung (wie unbehandelte Kontrolle).

pre-emergente Wirkung:

Konzentration der Wirkstoffemulsion: 70,8 ppm

| Testpflanze / Wirkstoff Nr | Nasturtium | Stellaria | Agrostis | Digitaria |
|---|---|---|---|---|
| 2.01 | 2 | 2 | 2 | 2 |
| 2.03 | 2 | 2 | 2 | 2 |
| 2.05 | 2 | 2 | 2 | 2 |
| 2.14 | 2 | 2 | 2 | 2 |
| 2.23 | 2 | 2 | 3 | 3 |
| 2.26 | 2 | 2 | 2 | 2 |
| 2.27 | 2 | 2 | 2 | 2 |
| 2.50 | 2 | 2 | 2 | 2 |
| 2.58 | 2 | 2 | 2 | 2 |
| 2.68 | 2 | 2 | 2 | 2 |
| 2.72 | 2 | 3 | 2 | 3 |
| 3.01 | 2 | 2 | 2 | 2 |
| 3.06 | 2 | 2 | 2 | 2 |
| 3.15 | 3 | 3 | 3 | 3 |
| 3.16 | 2 | 2 | 2 | 2 |
| 3.18 | 2 | 2 | 2 | 2 |
| 3.20 | 2 | 2 | 2 | 2 |
| 3.21 | 2 | 2 | 2 | 2 |
| 3.27 | 2 | 2 | 2 | 2 |

Beispiel 5: Wuchshemmung bei Getreide

In Kunststofftöpfen mit sterilisierter Erde werden in Getreidearten Hordeum vulgare (Sommergerste) und Secale (Sommerroggen) im Gewächshaus angesät und nach Bedarf bewässert. Die Sprösslinge werden ca. 21

Tage nach der Aussaat mit der wässrigen Spritzbrühe eines Wirkstoffes der Formel I besprüht. Die Wirkstoffmenge beträgt bis zu 100 g Aktivsubstanz pro Hektar. 21 Tage nach Applikation wird das Wachstum des Getreides beurteilt. Die behandelten Pflanzen weisen im Vergleich zu unbehandelten Kontrollen eine Verringerung des Neuzuwachses (60-90 % der Kontrolle), sowie teilweise eine Zunahme der Stengeldurchmesser auf.

Beispiel 6: Wuchshemmung bei Gräsern

In Kunststoffschalen mit Erde-Torf-Sand-Gemisch (6:3:1) werden die Gräser Lolium perenne, Poa pratensis, Festuca ovina, Dactylis glomerate und Cynodon dactylon im Gewächshaus angesät und nach Bedarf bewässert. Die aufgelaufenen Gräser werden wöchentlich bis auf 4 cm Höhe zurückgeschnitten und ca. 50 Tage nach der Aussaat und einen Tag nach dem letzten Schnitt mit der wässrigen Spritzbrühe eines Wirkstoffes der Formel I besprüht. Die Wirkstoffmenge beträgt umgerechnet bis zu 100 g Aktivsubstanz pro Hektar. 21 Tage nach Applikation wird das Wachstum der Gräser beurteilt.

Die Verbindungen der Formel I bewirken eine Reduzierung des Neuzuwachses von um 10-30 % im Vergleich zur unbehandelten Kontrolle.

**Patentansprüche**

1. N-Phenylsulfonyl-N′-pyrimidinyl-, N′-triazolyl- oder N′-triazin-yl-harnstoffe der Formel I

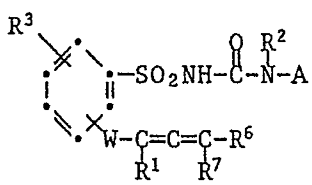

(I)

worin

$R^1$ und $R^2$ unabhängig voneinander je Wasserstoff oder $C_1$-$C_2$-Alkyl,

$R^3$ Wasserstoff, Halogen, Nitro, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkoxycarbonyl, $C_1$-$C_4$-Alkythio, $C_1$-$C_4$-Alkylsulfinyl, $C_1$-$C_4$-Alkylsulfonyl oder $C_2$-$C_5$-Alkoxyalkoxy oder $-So_2NR^4R^5$,

$R^4$ Wasserstoff oder $C_1$-$C_2$-Alkoxy oder $C_1$-$C_4$-Alkyl, $R^5$ Wasserstoff, Methyl oder Aethyl und

$R^6$ und $R^7$ je Wasserstoff, Methyl oder Aethyl

W Sauerstoff, Schwefel, eine Sulfinyl- oder Sulfonylbrücke und

A einen Rest

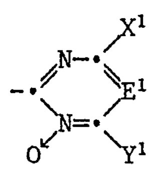

A1

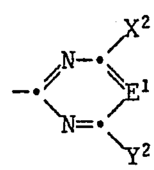

A2

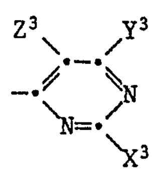

A3

,

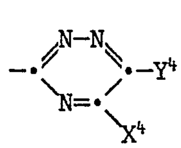

A4

,

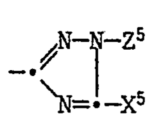

A5

,

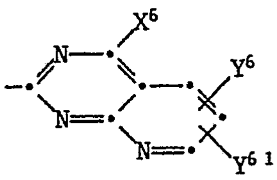

,

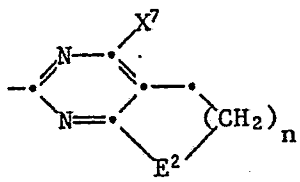

oder

A6

A7

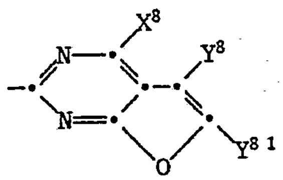

A8

bedeuten, wobei
$X^1$, $X^2$, $X^3$, $X^4$, $X^5$, $X^6$, $X^7$, $X^8$ und $Y^1$ unabhängig voneinander für Halogen, $C_1$-$C_4$-Halogenalkoxy, $C_1$-$C_4$-Halogenalkyl, $C_1$-$C_4$-Alkoxy, $C_1$-$C_4$-Alkyl, Dimethylamino, Methylamino, Aethylamino, Amino oder $C_2$-$C_4$-Alkoxyalkyl,
$E^1$ für Stickstoff oder die Methinbrücke,
$E^2$ für Sauerstoff oder die Methylenbrücke,
$Y^2$ für dasselbe wie $Y^1$ oder Cyclopropyl, Dimethoxymethyl, Diäthoxymethyl

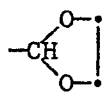

oder

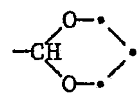

,

$Y^3$ für Methyl, Methoxy, Aethyl, Aethoxy, $C_1$-$C_2$-Halogenalkyl oder $C_1$-$C_2$-Halogenalkoxy,
$Y^4$ für Wasserstoff, Methoxy, Aethoxy, Halogen oder $C_1$-$C_4$-Alkyl,
$Y^6$ und $Y^{61}$ unabhängig voneinander für Wasserstoff, $C_1$-$C_4$-Alkyl, Halogen, Methoxy oder Methylthio,
$Y^8$ und $Y^{81}$ unabhängig voneinander für Wasserstoff oder Methyl,
n für eins oder zwei,
$Z^3$ für Wasserstoff, Methyl, Aethyl, Methoxy, Aethoxy, Methoxycarbonyl, Aethoxycarbonyl, Halogen, Cyan, Nitro, Methylthio, Methylsulfinyl oder Methylsulfonyl,
$Z^3$ und $Y^3$ zusammen für eine $C_2$-$C_4$-Alkylenbrücke oder eine durch Sauerstoff unterbrochene $C_2$-$C_4$-Alkylenbrücke, und
$Z^5$ für Methyl oder Aethyl stehen,
sowie den Salzen diesr Verbindungen.

2. N-Phenylsulfonyl-N'-pyrimidinyl-harnstoffe gemäss Anspruch 1 der Formel Ia

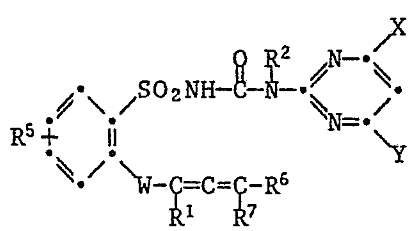

(Ib)

worin
$R^1$ und $R^2$ unabhängig voneinander je Wasserstoff oder $C_1$-$C_2$-Alkyl,
$R^3$ Wasserstoff, Halogen, Nitro, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylcarbonyl, $C_1$-$C_4$-Alkylthio, $C_1$-$C_4$-Alkylsulfinyl, $C_1$-$C_4$-Alkylsulfonyl, $C_2$-$C_5$-Alkoxyalkoxy oder $-SO_2NR^4R^5$,
$R^4$ Wasserstoff, $C_1$-$C_2$-Alkoxy
$R^5$ Wasserstoff, Methyl oder Aethyl
$R^6$ und $R^7$ Wasserstoff, Methyl oder Aethyl
W Sauerstoff, Schwefel, eine Sulfinyl- oder Sulfonylbrücke,

X Halogen, $C_1$-$C_4$-Alkyl, $C_1$-$C_4$-Halogenalkyl, $C_1$-$C_4$-Alkoxy, $C_1$-$C_4$-Halogenalkoxy, Amino, Methylamino, Aethylamino, Dimethylamino oder $C_2$-$C_5$-Alkoxyalkyl
Y dasselbe wie X oder Cyclopropyl
bedeuten.

3. N-Phenylsulfonyl-N'-pyrimidinyl-harnstoffe gemäss Anspruch 2 der Formel Ib, worin $R^1$, $R^2$, $R^3$, $R^6$ und $R^7$ je Wasserstoff und W Sauerstoff bedeutet und X und Y die im Anspruch 2 gegebene Bedeutung haben.

4. N-Phenylsulfonyl-N'-pyrimidinyl-harnstoffe gemäss Anspurch 2 der Formel Ib, worin $R^1$, $R^2$, $R^3$, $R^6$ und $R^7$ je Wasserstoff und W Schwefel bedeutet und X und Y die im Anspruch 2 gegebene Bedeutung haben.

5. N-(2-Allenyloxyphenylsulfonyl)-N'-(4-methoxy-4-methylpyrimidin-2-yl)-harnstoff gemäss Anspruch 1.

6. N-(2-Allenyloxyphenylsulfonyl)-N'-(4,6-dimethoxy-pyrimidin-2-yl)-harnstoff gemäss Anspruch 1.

7. N-(2-Allenyloxyphenylsulfonyl)-N'-(4,6-bis-difluoromethoxy-pyrimidin-2-yl)-harnstoff gemäss Anspruch 1.

8. N-Phenylsulfonyl)-N'-triazinyl-harnstoff gemäss Anspruch 1 der Formel Ic

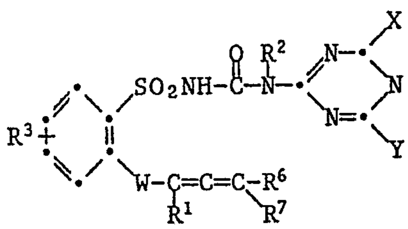

(Ic)

worin
$R^1$ und $R^2$ unabhängig voneinander je Wasserstoff oder $C_1$-$C_2$-Alkyl,
$R^3$ Wasserstoff, Halogen, Nitro, $C_1$-$C_4$-Alkyl, $C_1$-$C_4$-Halogenalkyl, $C_1$-$C_4$-Alkoxy, $C_1$-$C_4$-Haloalkoxy, $C_1$-$C_4$-Alkylcarbonyl, $C_1$-$C_4$-Alkylthio, $C_1$-$C_4$-Alkylsulfinyl, $C_1$-$C_4$-Alkylsulfonyl, $C_2$-$C_5$Alkoxyalkoxy oder $-SO_2NR^4R^5$,
$R^4$ Wasserstoff, $C_1$-$C_2$-Alkyl oder $C_1$-$C_2$-Alkoxy,
$R^5$ Wasserstoff, Methyl oder Aethyl
$R^6$ und $R^7$ Wasserstoff, Methyl oder Aethyl
W Sauerstoff, Schwefel, eine Sulfinyl- oder Sulfonylbrücke,
X Halogen, $C_1$-$C_4$-Alkyl, $C_1$-$C_4$Halogenalkyl, $C_1$-$C_4$-Alkoxy, $C_1$-$C_4$-Halogenalkoxy, Amino, Methylamino, Aethylamino, Dimethylamino oder $C_2$-$C_5$-Alkoxyalkyl und
Y dasselbe wie X oder Cyclopropyl
bedeuten.

9. N-(2-Allenyloxyphenylsulfonyl)-N'-triazinyl-harnstoff gemäss Anspruch 1 der Formel Ic worin $R^1$, $R^2$, $R^3$, $R^6$ und $R^7$ je Wasserstoff bedeutet und W, X und Y die im Anspruch 8 gegebene Bedeutung haben.

10. N-(2-Allenyloxyphenylsulfonyl)-N'-(4-methoxy-6-methyl-1,3,5-triazin-2-yl)-harnstoff gemäss Anspruch 1.

11. Verfahren zur Herstellung der N-Phenylsulfonylharnstoffe der Formel I, Anspruch 1, dadurch gekennzeichnet, dass man ein Phenylsulfonamid der Formel II

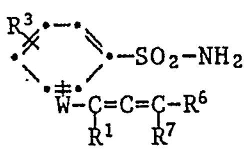

(II)

worin $R^1$, $R^3$, $R^6$, $R^7$ und W die im Anspruch 1 gegebene Bedeutung haben, in Gegenwart einer Base mit einem Carbamat der Formel III

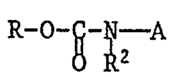

(III),

worin A und $R^2$ die im Anspruch 1 gegebene Bedeutung haben und R für Phenyl, Alkyl oder substituiertes Phenyl steht, umsetzt und gegebenenfalls in ein Salz überführt.

12. Verfahren zur Herstellung der Phenylsulfonylharnstoffe der Formel I, Anspruch 1, dadurch gekennzeichnet, dass man ein Sulfonylcarbamat der Formel IV

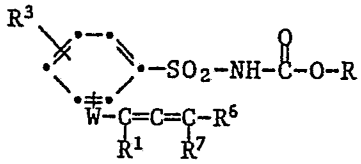 (IV),

worin $R^1$, $R^3$, $R^6$, $R^7$ und W die unter Formel I gegebene Bedeutung haben und R für Phenyl, Alkyl oder substituiertes Phenyl steht, mit einem Amin der Formel V

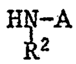 (V),

worin A und $R^2$ die unter Formel I gegebene Bedeutung haben, umsetzt und gegebenenfalls in ein Salz überführt.

13. Verfahren zur Herstellung der Phenylsulfonylharnstoffe der Formel I, Anspruch 1, dadurch gekennzeichnet, dass man ein Sulfonamid der Formel II

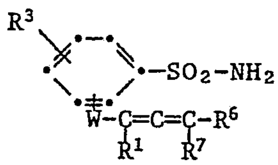 (II),

worin $R^1$, $R^3$, $R^6$, $R^7$ und W die im Anspruch 1 gegebene Bedeutung haben, mit einem Isocyanat der Formel VI

OCN-A (VI)

worin A die unter Formel I gegebene Bedeutung haben, umsetzt und gegebenenfalls in ein Salz überführt.

14. Verfahren zur Herstellung von Additionssalzen der Formel I gemäss einem der Ansprüche 10 bis 11, dadurch gekennzeichnet, dass man einen Sulfonylharnstoff der Formel I mit einem Amin, einem Alkalimetall- oder Erdalkalimetallhydroxid oder einer quaternären Ammoniumbase umsetzt.

15. Ein herbizides und den Pflanzenwuchs hemmendes Mittel, dadurch gekennzeichnet, dass es neben Träger- und/oder anderen Zuschlagstoffen als Wirkstoff einen Phenylsulfonylharnstoff der Formel I, Anspruch 1, enthält.

16. Verfahren zur Bekämpfung unerwünschten Pflanzenwachstums, dadurch gekennzeichnet, dass man einen Wirkstoff der Formel I, gemäss Anspruch 1, oder ein diesen Wirkstoff enthaltendes Mittel in einer wirksamen Menge auf die Pflanzen oder deren Lebensraum appliziert.

17. Verfahren zur Hemmung des Pflanzenwachstums, dadurch gekennzeichnet, dass man einen Wirkstoff der Formel I, gemäss Anspruch 1, oder ein diesen Wirkstoff enthaltendes Mittel in einer wirksamen Menge auf die Pflanzen oder deren Lebensraum appliziert.

18. Verfahren zur Beeinflussung des Pflanzenwachstums zum Zweck der Ertragssteigerung, dadurch gekennzeichnet, dass man einen Wirkstoff der Formel I, gemäss Anspruch 1, oder ein diesen Wirkstoff enthaltendes Mittel in einer wirksamen Menge auf die Pflanzen oder deren Lebensraum appliziert.

19. Verfahren gemäss Anspruch 15, zur selektiven pre- oder postemergenten Bekämpfung von Unkräutern in Nutzpflanzenkulturen.

20. Verfahren gemäss Anspruch 16, zur Unterdrückung des Pflanzenwachstums über das 2-Blattstadium hinaus, dadurch gekennzeichnet, dass die Wirkstoffe preemergent angewendet werden.